(19) Europäisches Patentamt
European Patent Office
Office européen des brevets

(11) EP 1 064 916 A1

(12) **DEMANDE DE BREVET EUROPEEN**

(43) Date de publication:
**03.01.2001 Bulletin 2001/01**

(51) Int Cl.[7]: **A61K 7/06**

(21) Numéro de dépôt: **00401425.4**

(22) Date de dépôt: **23.05.2000**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **25.06.1999 FR 9908173**

(71) Demandeur: **L'OREAL**
**75008 Paris (FR)**

(72) Inventeurs:
- **Dubief, Claude**
**78150 Le Chesnay (FR)**
- **Restle, Serge**
**95390 Saint-Prix (FR)**

(74) Mandataire: **Dodin, Catherine**
**L'OREAL-DPI**
**6 rue Bertrand Sincholle**
**92585 Clichy Cédex (FR)**

(54) **Compositions cosmétiques contenant un polymère amphotère et un agent antipelliculaire et leurs utilisations**

(57) L'invention concerne de nouvelles compositions cosmétiques comprenant dans un milieu cosmétiquement acceptable au moins un agent antipelliculaire et au moins un polymère amphotère comprenant au moins un monomère de type (méth)acrylate ou (méth) acrylamide ayant au moins une chaîne grasse, ladite chaîne grasse ayant de 8 à 30 atomes de carbone.

Cette association permet d'améliorer le dépôt de l'agent antipelliculaire.

Ces compositions sont utilisées pour le lavage et/ ou le conditionnement des matières kératiniques telles que les cheveux ou la peau.

EP 1 064 916 A1

## Description

La présente invention concerne de nouvelles compositions cosmétiques comprenant dans un milieu cosmétiquement acceptable au moins un agent antipelliculaire et au moins un polymère amphotère comprenant au moins un monomère de type (méth)acrylate ou (méth)acrylamide ayant au moins une chaîne grasse, ladite chaîne grasse ayant de 8 à 30 atomes de carbone.

En vue de combattre la formation des pellicules qui est généralement accompagnée d'une prolifération microbienne et/ou fongique, il a été proposé comme produits antipelliculaires soit des produits inhibant la prolifération microbienne, soit des produits kératolytiques.

Cependant, les cheveux traités avec les agents antipelliculaires présentent un toucher rêche et chargé. De plus, le démêlage est relativement difficile.

En outre, l'activité antifongique (en particulier vis-à-vis de Malassezia Ovalis responsable de la formation des pellicules) est encore insuffisante.

La présente invention a donc pour but de proposer des compositions permettant de lutter efficacement contre les pellicules tout en apportant de bonnes propriétés en particulier de douceur et de démêlage des cheveux.

On a déjà préconisé dans les compositions pour le lavage ou le soin des matières kératiniques telles que les cheveux l'utilisation d'agents conditionneurs, notamment des polymères cationiques ou amphotères ou des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légèreté du cheveu), un manque de lissage (cheveu non homogène de la racine à la pointe).

En outre, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage. Ces inconvénients sont accentués dans le cas de cheveux fins, qui manquent de nervosité et de volume.

En résumé, il s'avère que les compositions cosmétiques actuelles contenant des agents antipelliculaire, ne donnent pas complètement satisfaction.

La demanderesse a maintenant découvert que l'association d'un polymère amphotère particulier avec un agent antipelliculaire permet de remédier à ces inconvénients.

Ainsi, à la suite d'importantes recherches menées sur la question, il a maintenant été trouvé par la Demanderesse qu'en introduisant un polymère amphotère particulier dans les compositions en particulier capillaires à base d'agents antipelliculaires, il est possible d'augmenter le dépôt de l'agent antipelliculaire, tout en améliorant les propriétés cosmétiques des compositions à base d'agents antipelliculaires.

Sans vouloir limiter la présente invention à une quelconque théorie, il semblerait exister lors du rinçage, des interactions et/ou des affinités particulières entre l'agent antipelliculaire, les polymères amphotères conformes à l'invention et les cheveux, qui favorisent un dépôt régulier, important et durable desdites agents antipelliculaires et polymères amphotères à la surface desdits cheveux, ce dépôt qualitatif et quantitatif étant probablement l'une des causes de l'amélioration observée au niveau des propriétés finales, en particulier la facilité de coiffage, le maintien, la nervosité et le volume des cheveux traités. Toutes ces découvertes sont à la base la présente invention.

Les compositions conformes à l'invention confèrent aux matières kératiniques notamment les cheveux, un remarquable effet traitant qui se manifeste notamment par une facilité de démêlage, ainsi qu'un apport de volume, de légèreté, de lissage, de douceur et de souplesse et de discipline sans aucune sensation de toucher chargé.

Ainsi, selon la présente invention, il est maintenant proposé de nouvelles compositions cosmétiques, comprenant, dans un milieu cosmétiquement acceptable, au moins un agent antipelliculaire et au moins un polymère amphotère comprenant au moins un monomère de type (méth)acrylate ou (méth)acrylamide ayant au moins une chaîne grasse, ladite chaîne grasse ayant de 8 à 30 atomes de carbone.

Un autre objet de l'invention concerne l'utilisation d'un polymère amphotère comprenant au moins un monomère de type (méth)acrylate ou (méth)acrylamide ayant au moins une chaîne grasse, ladite chaîne grasse ayant de 8 à 30 atomes de carbone dans, ou pour la fabrication d'une composition cosmétique comprenant un agent antipelliculaire.

L'invention a également pour objet l'utilisation d'un polymère amphotère comprenant au moins un monomère de type (méth)acrylate ou (méth)acrylamide ayant au moins une chaîne grasse, ladite chaîne grasse ayant de 8 à 30 atomes de carbone dans une composition comprenant un agent antipelliculaire pour augmenter l'efficacité de cet agent antipelliculaire.

Les différents objets de l'invention vont maintenant être détaillés. L'ensemble des significations et définitions des composés utilisés dans la présente invention données ci-dessous sont valables pour l'ensemble des objets de l'invention.

Les polymères amphotères selon l'invention comprennent généralement de 1 à 20 moles% de monomère comportant une chaîne grasse, et de préférence de 1,5 à 15 moles % par rapport au nombre total de moles de monomères et plus particulièrement de 1,5 à 6 moles %.

Les polymères amphotères selon l'invention peuvent résulter de la copolymérisation

1) d'au moins un monomère de formule (Ia) ou (Ib):

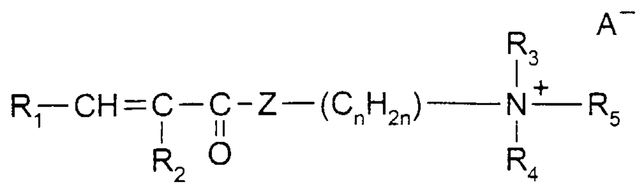

(Ia)

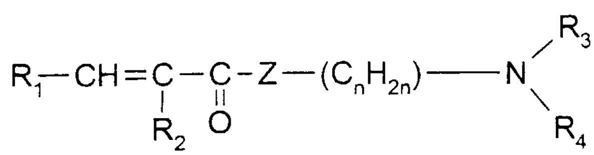

(Ib)

dans lesquelles $R_1$ et $R_2$, identiques ou différents, représentent un atome d'hydrogène ou un radical méthyle, $R_3$, $R_4$ et $R_5$, identiques ou différents, représente un radical alkyle linéaire ou ramifié ayant de 1 à 30 atomes de carbone,

Z représente un groupe NH ou un atome d'oxygène,
n est un nombre entier de 2 à 5,
$A^-$ est un anion issu d'un acide organique ou minéral, tel qu'un anion méthosulfate ou un halogénure tel que chlorure ou bromure.

2) d'au moins un monomère de formule (II)

$$R_6-CH=CR_7-COOH \quad (II)$$

dans laquelle $R_6$ et $R_7$, identiques ou différents, représentent un atome d'hydrogène ou un radical méthyle;
et
3) d'au moins un monomère de formule (III):

$$R_6-CH=CR_7-COXR_8 \quad (III)$$

dans laquelle $R_6$ et $R_7$, identiques ou différents, représentent un atome d'hydrogène ou un radical méthyle, X désigne un atome d'oxygène ou d'azote et $R_8$ désigne un radical alkyle linéaire ou ramifié ayant de 1 à 30 atomes de carbone;

l'un au moins des monomères de formule (Ia), (Ib) ou (III) comportant au moins une chaîne grasse ayant de 8 à 30 atomes de carbone.

Les monomères de formule (Ia) et (Ib) de la présente invention sont choisis, de préférence, dans le groupe constitué par :

- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,

- le diméthylaminopropylméthacrylamide, le diméthylaminopropylacrylamide,

ces monomères étant éventuellement quaternisés par exemple par un halogénure d'alkyle en $C_1$-$C_4$ ou un sulfate de dialkyle en $C_1$-$C_4$.

Plus particulièrement, le monomère de formule (Ia) est choisi parmi le chlorure d'acrylamidopropyl triméthyl ammonium et le chlorure de méthacrylamidopropyl triméthyl ammonium.

Les monomères de formule (II) de la présente invention sont choisis, de préférence, dans le groupe constitué par l'acide acrylique, l'acide méthacrylique, l'acide crotonique et l'acide méthyl-2 crotonique.
Plus particulièrement, le monomère de formule (II) est l'acide acrylique.

Les monomères de formule (III) de la présente invention sont choisis, de préférence, dans le groupe constitué des acrylates ou méthacrylate d'alkyle en $C_{12}$-$C_{22}$ et plus particulièrement en $C_{16}$-$C_{18}$.

Les monomères constituant les polymères amphotères de l'invention sont de préférence déjà neutralisés et/ou quaternisés.

Le rapport du nombre de charges cationiques/charges anioniques est de préférence égal à environ 1.

Les polymères amphotères selon l'invention comprennent de préférence de 1 à 10 moles% du monomère comportant une chaîne grasse (monomère de formule (Ia), (Ib) ou (III)), et de préférence de 1,5 à 6 moles % .

Les poids moléculaires moyen en poids des polymères amphotères selon l'invention peuvent varier de 500 à 50.000.000 et sont de préférence compris entre 10.000 et 5 000 000.

Les polymères selon l'invention peuvent également contenir d'autre monomères tels que des monomères non ioniques et en particulier tels que les acrylates ou méthacrylates d'alkyle en $C_1$-$C_4$.

Les polymères amphotères selon l'invention sont par exemple décrits dans la demande de brevet WO9844012.

Les polymères amphotères particulièrement préférés selon l'invention sont choisis parmi les copolymères acide acrylique/chlorure d'acrylamidopropyl triméthyl ammonium/ méthacrylate de stéaryle.

Le polymère amphotère est utilisé de préférence en une quantité comprise entre 0,05 et 10% en poids par rapport au poids total de la composition. Plus préférentiellement, cette quantité est comprise entre 0,1 et 5% en poids par rapport au poids total de la composition.

Les agents antipelliculaires peuvent être tout agent actif utile pour prévenir l'apparition des pellicules, diminuer leur nombre et/ou les faire disparaître totalement. Ainsi, l'agent antipelliculaire peut être choisi parmi les agents antifongiques et/ou antibactériens.

Les agents antipelliculaires utilisables selon l'invention sont notamment choisis parmi :

1) les sels de pyridinethione notamment les sels de calcium, de magnésium, de barium, de strontium, de zinc, de cadmium, d'étain et de zirconium. Le sel de zinc de pyridinethione est particulièrement préféré.
Le sel de zinc de pyridinethione est notamment commercialisé sous la dénomination Omadine de zinc par la société OLIN.

2) les dérivés de 1-hydroxy-2-pyrrolidone représentés par la formule (IV) ;

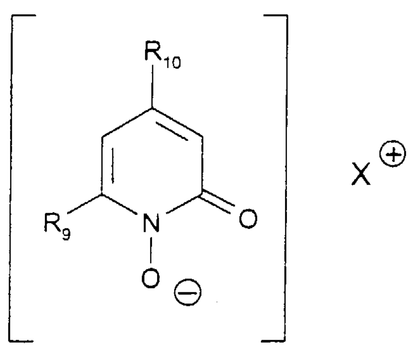

(IV)

dans laquelle R9 représente un groupe alkyle ayant de 1 à 17 atomes de carbone, alkényle ayant de 2 à 17 atomes de carbone, un groupe cycloalkyle ayant de 5 à 8 atomes de carbone, un groupe bicycloalkyle ayant de 7 à 9 atomes de carbone ; un groupe cycloalkyl (-alkyle ), un groupe aryle, un groupe aralkyle avec un alkyle ayant de 1 à 4 atome de carbone, un groupe arylalkényl avec un alkényle ayant de 2 à 4 atome de carbone, aryloxyalkyle ou arylmercaptoalkyle avec un alkyle ayant de 1 à 4 atome de carbone, un groupe furylalkényl avec un alkényle

ou un furyle ayant de 2 à 4 atome de carbone, un groupe alkoxy ayant de 1 à 4 atome de carbone, un groupe nitro, un groupe cyano ou un atome d'halogène.

R10 représente un atome d'hydrogène, un groupe alkyle en C1-C4, un groupe alkényle en C2-C4, un atome d'halogène, un groupe phényle , un groupe benzyle ; Y représente une base organique, un ion de métal alcalin ou alcalinoterreux, un ion ammonium.

Des composés de formules (IV) sont par exemple le 1-hydroxy-4-méthyl-2-pyridone, le 1-hydroxy-6-méthyl-2-pyridone, le 1-hydroxy-4,6-diméthyl-2-pyridone, le 1-hydroxy-4-méthyl-6-(2,4,4-triméthylpentyl)-2-pyridone, le 1-hydroxy-4-méthyl-6-cyclohexyl-2-pyridone, le 1-hydroxy-4-méthyl-6-(méthyl-cyclohexyl)-2-pyridone, le 1-hydroxy-4-méthyl-6-(2-bicyclo[2,2,1]heptyl)-2-pyridone, le 1-hydroxy-4-methyl-6-(4-methyl-phenyl)-2-pyridone, le 1-hydroxy-4-methyl-6-[1-(4-nitrophenoxy)-butyl]-2-pyridone, le 1-hydroxy-4-méthyl-6-(4-cyanophenoxymethyl)-2-pyridone, le 1-hydroxy-4-methyl-6-(phenylsulfonylmethyl)-2-pyridone, le 1-hydroxy-4méthyl-6-(4-bromo-benzyl)-2-pyridone.

Les composés de formule (IV) peuvent être utilisés sous forme de sels avec des bases organiques ou inorganiques.

Des exemples de bases organiques sont notamment les alkanol amines de faibles poids moléculaires tels que l'éthanolamine, la diéthanolamine, la N-éthyléthanolamine, la triéthanolamine, le diéthylaminoéthanol, le 2-amino-2-méthylpropanediol; les bases non-volatile telles que l'éthylènediamine, l'hexaméthylènediamine, la cyclohexylamine, la benzylamine, la N-méthylpipérazine; les hydroxydes d'ammonium quaternaires tels que le triméthylbenzyl hydroxyde; la guanidine et ses dérivés, et particulièrement ses dérivés alkylés. Des exemples de bases inorganiques sont notamment les sels de métaux alcalins tels que sodium, potassium; les sels d'ammonium, les sels de métaux alcalinoterreux, tels que magnésium, calcium; les sels de métaux di, tri ou tétravalents cationiques, tels que zinc, aluminium, zirconium. Les alkanolamines, l'éthylènediamine; et les bases inorganiques telles que les sels de métal alcalin sont préférés.

Un composé de formule (IV) particulièrement préféré est celui pour lequel

$R_9$ désigne le radical

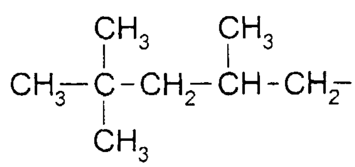

$R_{10}$ désigne méthyle
et $X^+$ désigne $N^+H_3CH_2CH_2OH$

Ce composé est par exemple commercialisé sous la dénomination Octopirox (1-hydroxy-4-méthyl-6-(2,4,4-triméthylpentyl)-2-pyridone, sel de monoéthanolamine) par la société HOECHST.

3) le 2,2'-dithio-bis-(pyridine-N-oxide) de formule (V):

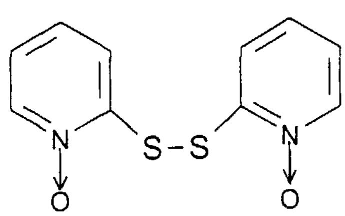

(V)

Les composés de formule (V) peuvent être introduit dans les compositions sous forme de sels inorganiques. Des exemples de sels inorganiques sont le sulfate de magnésium.

4) les trihalogéno carbamide de formule (VI) suivante :

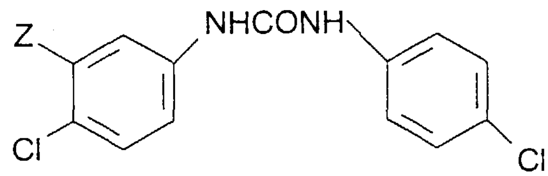

(VI)

dans laquelle Z représente un atome d'halogène comme le chlore ou un groupement trihalogénoalkyle en C1-C4 tel que CF3.

5) le triclosan représenté par la formule (VII)

(VII)

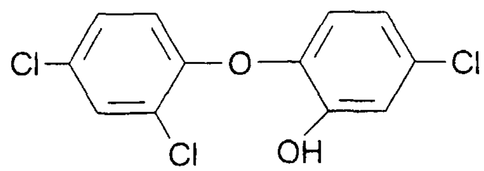

6) les composés azolés tels que le climbazole, le kétoconazole, le clotrinazole, l'econazole, l'isoconazole et le miconazole.

7) les polymères antifongiques tels que l'amphotéricine B ou la nystatine.

8) le sulfure de sélénium

9) D'autres agents antipelliculaires, sont le soufre sous ses différentes formes, le sulfure de cadmium, l'allantoïne, les goudrons de houille ou de bois et leurs dérivés en particulier l'huile de cade, l'acide salicylique, l'acide undécylénique, l'acide fumarique, les allylamines telle que la terbinafine.

L'Omadoine de Zinc, l'Octopirox et le sulfure de sélénium sont particulièrement préférés.

Selon l'invention, le ou les agents antipelliculaires peuvent représenter de 0,001 % à 20 % en poids, de préférence de 0,01 % à 10% en poids et plus particulièrement de 0,1 à 5% en poids par rapport au poids total de la composition finale.

Les compositions de l'invention contiennent en outre avantageusement au moins un agent tensioactif qui est généralement présent en une quantité comprise entre 0,1% et 60% en poids environ, de préférence entre 3% et 40% et encore plus préférentiellement entre 5% et 30%, par rapport au poids total de la composition.

Cet agent tensioactif peut être choisi parmi les agents tensioactifs anioniques, amphotères, non-ioniques, ou leurs mélanges.

Les tensioactifs convenant à la mise en oeuvre de la présente invention sont notamment les suivants :

(i) Tensioactif(s) anionique(s) :

Leur nature ne revêt pas, dans le cadre de la présente invention, de caractère véritablement critique. Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, a-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique,

stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl ($C_6$-$C_{24}$) éther carboxyliques polyoxyalkylénés, les acides alkyl($C_6$-$C_{24}$)aryl éther carboxyliques polyoxyalkylénés ,les acides alkyl($C_6$-$C_{24}$) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

(ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine. On notera que les alkylpolyglycosides constituent des tensioactifs nonioniques rentrant particulièrement bien dans le cadre de la présente invention.

(iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) bétaïnes ou les alkyl ($C_8$-$C_{20}$) amidoalkyl ($C_1$-$C_6$) sulfobétaïnes.

Parmi les dérivés d'amines, on peut citer les produits commercialisés sous les dénomination MIRANOL, tels que décrits dans les brevets US-2 528 378 et US-2 781 354 et de structures :

$$R_2\text{-CONHCH}_2\text{CH}_2\text{-N}(R_3)(R_4)(\text{CH}_2\text{COO-}) \quad (2)$$

dans laquelle : $R_2$ désigne un radical alkyle dérivé d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ;
et

$$R_5\text{-CONHCH}_2\text{CH}_2\text{-N(B)(C)} \quad (3)$$

dans laquelle :

B représente $-CH_2CH_2OX'$, C représente $-(CH_2)_z$ -Y', avec z = 1 ou 2,
X' désigne le groupement $-CH_2CH_2$-COOH ou un atome d'hydrogène
Y' désigne -COOH ou le radical $-CH_2$ - CHOH - $SO_3H$
$R_5$ désigne un radical alkyle d'un acide $R_9$ -COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHONE POULENC.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence un agent tensioactif anionique choisi parmi les alkyl($C_{12}$-$C_{14}$) sulfates de sodium, de triéthanolamine ou d'ammonium, les alkyl ($C_{12}$-$C_{14}$)éthersulfates de sodium, de triéthanolamine ou d'ammonium oxyéthylénés à 2,2 moles d'oxyde d'éthylène, le cocoyl iséthionate de sodium et l'alphaoléfine($C_{14}$-$C_{16}$) sulfonate de sodium et leurs mélange avec :

- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société HENKEL.

La composition de l'invention peut également contenir au moins un additif choisi parmi les épaississants, les parfums, les agents nacrants, les conservateurs, les filtres solaires siliconés ou non, les vitamines, les cires minérales, les polymères cationiques, anioniques ou non ioniques, les protéines, les hydrolysats de protéines, les acides gras à chaînes linéaires ou ramifiées en $C_{16}$-$C_{40}$ tels que l'acide méthyl-18 eicosanoique, les esters d'acide gras, les tensioactifs cationiques; les alcools gras, les hydroxyacides, les vitamines, le panthénol, les huiles animales, les huiles minérales, les huiles de synthèse, les huiles végétales, les composés de type céramide et tout autre additif classiquement utilisé dans le domaine cosmétique qui n'affecte pas les propriétés des compositions selon l'invention.

Ces additifs sont présents dans la composition selon l'invention dans des proportions pouvant aller de 0 à 20% en poids par rapport au poids total de la composition. La quantité précise de chaque additif est déterminée facilement par l'homme du métier selon sa nature et sa fonction.

Les compositions conformes à l'invention peuvent être plus particulièrement utilisées pour le lavage ou le traitement des matières kératiniques telles que les cheveux. Elles peuvent être utilisées sur le cuir chevelu, la peau, les cils, les sourcils, les ongles, les lèvres.

En particulier, les compositions selon l'invention sont des compositions détergentes telles que des shampooings, des gels-douche et des bains moussants. Dans ce mode de réalisation de l'invention, les compositions comprennent une base lavante, généralement aqueuse.

Le ou les tensioactifs formant la base lavante peuvent être indifféremment choisis, seuls ou en mélanges, au sein des tensioactifs anioniques, amphotères, non ioniques et cationiques tels que définis ci-dessus.

La quantité et la qualité de la base lavante sont celles suffisantes pour conférer à la composition finale un pouvoir moussant et/ou détergent satisfaisant.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits. Dans les exemples, MA signifie matière active.

## EXEMPLE

On a préparé le shampooing de composition suivante :

| | |
|---|---|
| Lauryléther sulfate de sodium (2,2 OE) | 17 g MA |
| Cocoyl bétaïne en solution aqueuse à 30% (DEHYTON AB 30 de HENKEL) | 2,5 g MA |
| Terpolymère de chlorure de méthacrylamidopropyl triméthyl ammonium, d'acide acrylique et de méthacrylate de stéaryle (49% / 49% / 2% molaire) | 1 g MA |
| Cétostéaryl sulfate de sodium | 0,75 g |
| Monoisopropanolamide d'acides de coprah | 0,6 g |
| 1-hydroxy 4-méthyl-6-triméthylpentyl-2-pyridone (OCTOPIROX de CLARIANT) | 0,5 gMA |
| Conservateur,parfum | q. s |
| Eau q. s. p | 100 g |

## Revendications

1. Composition cosmétique, caractérisée par le fait qu'elle comprend, dans un milieu cosmétiquement acceptable, au moins un agent antipelliculaire et au moins un polymère amphotère comprenant au moins un monomère de type (méth)acrylate ou (méth)acrylamide ayant au moins une chaîne grasse, ladite chaîne grasse ayant de 8 à 30 atomes de carbone.

2. Composition selon la revendication 1, caractérisée par le fait que ledit polymère amphotères comprend de 1 à 20 moles% de monomère comportant une chaîne grasse, et de préférence de 1,5 à 15 moles % par rapport au nombre total de moles de monomères.

3. Composition selon l'une quelconque des revendications 1 ou 2, caractérisée par le fait les polymères amphotères résultent de la copolymérisation

   1) d'au moins un monomère de formule (Ia) ou (Ib):

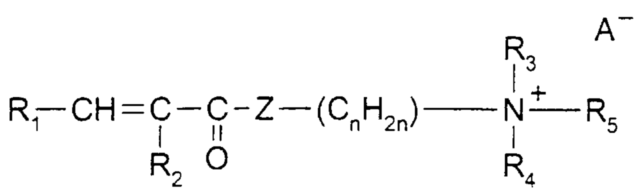

(Ia)

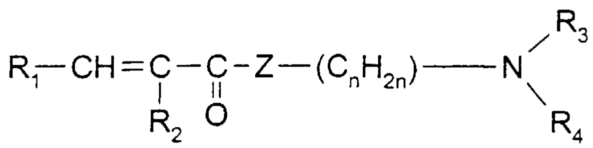 (Ib)

dans lesquelles $R_1$ et $R_2$, identiques ou différents, représentent un atome d'hydrogène ou un radical méthyle, $R_3$, $R_4$ et $R_5$, identiques ou différents, représente un radical alkyle linéaire ou ramifié ayant de 1 à 30 atomes de carbone,

Z représente un groupe NH ou un atome d'oxygène,
n est un nombre entier de 2 à 5,
$A^-$ est un anion issu d'un acide organique ou minéral.

2) d'au moins un monomère de formule (II)

$$R_6-CH=CR_7-COOH \quad (II)$$

dans laquelle $R_6$ et $R_7$, identiques ou différents, représentent un atome d'hydrogène ou un radical méthyle; et
3) d'au moins un monomère de formule (III):

$$R_6-CH=CR_7-COXR_8 \quad (III)$$

dans laquelle $R_6$ et $R_7$, identiques ou différents, représentent un atome d'hydrogène ou un radical méthyle, X désigne un atome d'oxygène ou d'azote et $R_8$ désigne un radical alkyle linéaire ou ramifié ayant de 1 à 30 atomes de carbone;

l'un au moins des monomères de formule (Ia), (Ib) ou (III) comportant au moins une chaîne grasse ayant de 8 à 30 atomes de carbone.

**4.** Composition selon la revendications 3, caractérisée par le fait que les monomères de formule (Ia) et (Ib) de la présente invention sont choisis dans le groupe constitué par :

- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide, ces monomères étant éventuellement quaternisés

**5.** Composition selon l'une quelconque des revendications 3 ou 4, caractérisée par le fait que le monomère de formule (Ia) est choisi parmi le chlorure d'acrylamidopropyl triméthyl ammonium et le chlorure de méthacrylamidopropyl triméthyl ammonium.

**6.** Composition selon l'une quelconque des revendications 3 à 5, caractérisée par le fait que les monomères de formule (II) sont choisis dans le groupe constitué par l'acide acrylique, l'acide méthacrylique, l'acide crotonique et l'acide méthyl-2 crotonique.

**7.** Composition selon l'une quelconque des revendications 3 à 6, caractérisée par le fait que les monomères de formule (III) sont choisis dans le groupe constitué des acrylates ou méthacrylate d'alkyle en $C_{12}$-$C_{22}$ et plus particulièrement en $C_{16}$-$C_{18}$.

**8.** Composition selon l'une quelconque des revendications 1 à 7, caractérisée par le fait que lesdits polymères amphotères sont choisis parmi les copolymères acide acrylique/chlorure d'acrylamidopropyl triméthyl ammonium/méthacrylate de stéaryle.

**9.** Composition selon l'une quelconque des revendications précédentes, caractérisée par le fait que ledit polymère amphotère est utilisé dans les compositions selon l'invention en une quantité comprise entre 0,05 et 10% en poids par rapport au poids total de la composition.

**10.** Composition selon l'une quelconque des revendications précédentes, caractérisée par le fait que ledit agent antipelliculaire est choisi parmi les agents antifongiques et/ou antibactériens.

**11.** Composition selon la revendication précédente, caractérisée par le fait que ledit agent antipelliculaire est choisi parmi

1) les sels de pyridinethione notamment les sels de calcium, de magnésium, de barium, de strontium, de zinc, de cadmium, d'étain et de zirconium.
2) les dérivés de 1-hydroxy-2-pyrrolidone représentés par la formule (IV) ;

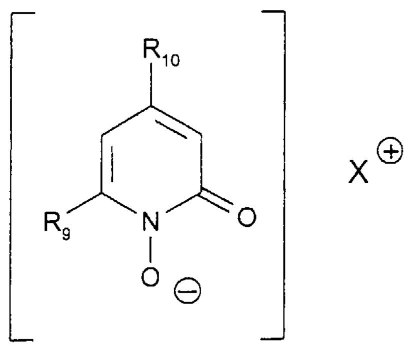

(IV)

dans laquelle R9 représente un groupe alkyle ayant de 1 à 17 atomes de carbone, alkényle ayant de 2 à 17 atomes de carbone, un groupe cycloalkyle ayant de 5 à 8 atomes de carbone, un groupe bicycloalkyle ayant de 7 à 9 atomes de carbone ; un groupe cycloalkyl (-alkyle ), un groupe aryle, un groupe aralkyle avec un alkyle ayant de 1 à 4 atome de carbone, un groupe arylalkényl avec un alkényle ayant de 2 à 4 atome de carbone, aryloxyalkyle ou arylmercaptoalkyle avec un alkyle ayant de 1 à 4 atome de carbone, un groupe furylalkényl avec un alkényle ou un furyle ayant de 2 à 4 atome de carbone, un groupe alkoxy ayant de 1 à 4 atome de carbone, un groupe nitro, un groupe cyano ou un atome d'halogène.
R10 représente un atome d'hydrogène, un groupe alkyle en C1-C4, un groupe alkényle en C2-C4, un atome d'halogène, un groupe phényle , un groupe benzyle ; Y représente une base organique, un ion de métal alcalin ou alcalinoterreux, un ion ammonium
3) le 2,2'-dithio-bis-(pyridine-N-oxide) de formule (V):

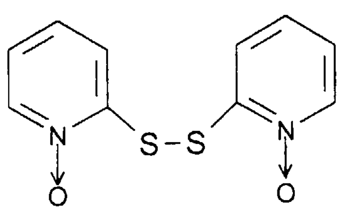

(V)

4) les trihalogéno carbamide de formule (VI) suivante :

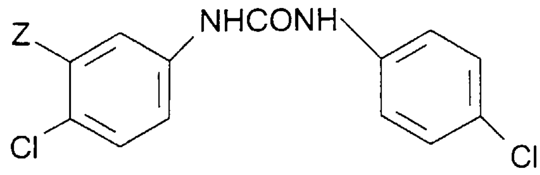

(VI)

dans laquelle Z représente un atome d'halogène comme le chlore ou un groupement trihalogénoalkyle en C1-C4 tel que CF3.

5) le triclosan représenté par la formule (VII)

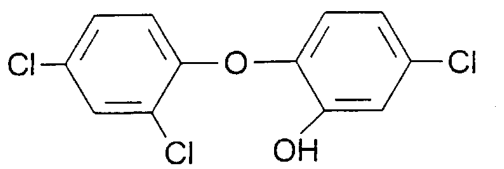

(VII)

6) les composés azolés tels que le climbazole, le kétoconazole, le clotrinazole, l'econazole, l'isoconazole et le miconazole.

7) les polymères antifongiques tels que l'amphotéricine B ou la nystatine.

8) le sulfure de sélénium

le soufre sous ses différentes formes, le sulfure de cadmium, l'allantoïne, les goudrons de houille ou de bois et leurs dérivés en particulier l'huile de cade, l'acide salicylique, l'acide undécylénique, l'acide fumarique, les allylamines telle que la terbinafine.

**12.** Composition selon la revendication précédente, caractérisée par le fait que ledit agent antipelliculaire est choisi parmi :

- le sel de zinc de pyridinethione
- le 1-hydroxy-4-méthyl-6-(2,4,4-triméthylpentyl )-2-pyridone
- le sulfure de sélénium.

**13.** Composition selon l'une quelconque des revendications précédentes, caractérisée en ce que l'agent antipelliculaire est présent à une concentration comprise entre 0,001 % et 20 % en poids par rapport au poids total de la composition, de préférence entre 0,01 % et 10 % en poids.

**14.** Composition selon l'une quelconque des revendications précédentes, caractérisée par le fait qu'elle comprend en outre au moins un agent tensioactif.

**15.** Composition selon la revendication 14, caractérisée par le fait que le ou les agents tensioactifs sont présents à une concentration comprise entre 0,1% et 60% en poids, de préférence entre 3% et 40% en poids, par rapport au poids total de la composition.

**16.** Composition selon l'une quelconque des revendications précédentes, caractérisée par le fait qu'elle comprend en outre au moins un additif choisi parmi les épaississants, les parfums, les agents nacrants, les conservateurs, les filtres solaires siliconés ou non, les vitamines, les cires minérales, les polymères cationiques, anioniques ou non ioniques, les protéines, les hydrolysats de protéines, les acides gras à chaînes linéaires ou ramifiées en $C_{16}$-$C_{40}$ tels que l'acide méthyl-18 eicosanoique, les esters d'acide gras, les alcools gras, les hydroxyacides, les vitamines, le panthénol, les tensioactifs cationiques, les huiles animales, les huiles minérales, les huiles de synthèse, les huiles végétales, les composés de type céramide et tout autre additif classiquement utilisé dans le domaine cosmétique.

**17.** Composition selon l'une quelconque des revendications précédentes, caractérisée par le fait qu'elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition pour la peau.

**18.** Utilisation d'une composition telle que définie dans l'une quelconque des revendications précédentes pour le lavage ou pour le soin des matières kératiniques.

**19.** Procédé de traitement des matières kératiniques, telles que les cheveux, caractérisé en ce qu'il consiste à appliquer sur lesdites matières une composition cosmétique selon l'une des revendications 1 à 17, puis à effectuer éventuellement un rinçage à l'eau.

**20.** Utilisation d'un polymère amphotère comprenant au moins un monomère de type (méth)acrylate ou (méth)acrylamide ayant au moins une chaîne grasse, ladite chaîne grasse ayant de 8 à 30 atomes de carbone dans une composition comprenant un agent antipelliculaire pour augmenter l'efficacité de cet agent antipelliculaire.

**21.** Utilisation d'un polymère amphotère à chaîne grasse tel que défini à l'une des revendications 1 à 8 dans, ou pour la fabrication d'une composition cosmétique comprenant un agent antipelliculaire.

Office européen des brevets

# RAPPORT DE RECHERCHE EUROPEENNE

Numéro de la demande
EP 00 40 1425

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | US 4 402 977 A (GROLLIER JEAN F ET AL) 6 septembre 1983 (1983-09-06) --- | | A61K7/06 |
| A | DE 44 04 493 C (KAO CORP GMBH) 19 janvier 1995 (1995-01-19) --- | | |
| D,A | WO 98 44012 A (CALGON CORP) 8 octobre 1998 (1998-10-08) ----- | | |

DOMAINES TECHNIQUES RECHERCHES (Int.Cl.7)
A61K

Le présent rapport a été établi pour toutes les revendications

| Lieu de la recherche | Date d'achèvement de la recherche | Examinateur |
|---|---|---|
| LA HAYE | 19 octobre 2000 | Stienon, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 00 40 1425

La présente annexe indique les membres de la famille de brevets relatifs aux documents brevets cités dans le rapport de recherche européenne visé ci-dessus.
Lesdits members sont contenus au fichier informatique de l'Officeeuropéen des brevets à la date du
Les renseignements fournis sont donnés à titre indicatif et n'engagent pas la responsabilité de l'Office européen des brevets.

19-10-2000

| Document brevet cité au rapport de recherche | Date de publication | Membre(s) de la famille de brevet(s) | Date de publication |
|---|---|---|---|
| US 4402977 A | 06-09-1983 | FR 2486394 A | 15-01-1982 |
| | | AT 378912 B | 25-10-1985 |
| | | AT 580780 A | 15-03-1985 |
| | | BE 886372 A | 27-05-1981 |
| | | CA 1158560 A | 13-12-1983 |
| | | CH 650669 A | 15-08-1985 |
| | | DE 3044754 A | 19-06-1981 |
| | | DE 3051202 C | 14-05-1992 |
| | | DK 100292 A | 10-08-1992 |
| | | DK 507680 A | 29-05-1981 |
| | | ES 497178 D | 01-05-1982 |
| | | ES 8204598 A | 16-08-1982 |
| | | GB 2088209 A,B | 09-06-1982 |
| | | IT 1129379 B | 04-06-1986 |
| | | JP 1823923 C | 10-02-1994 |
| | | JP 3021524 B | 22-03-1991 |
| | | JP 56092813 A | 27-07-1981 |
| | | JP 1188547 A | 27-07-1989 |
| | | JP 3075526 B | 02-12-1991 |
| | | NL 8006460 A | 01-07-1981 |
| | | SE 461131 B | 15-01-1990 |
| | | SE 8008333 A | 29-05-1981 |
| DE 4404493 C | 19-01-1995 | AUCUN | |
| WO 9844012 A | 08-10-1998 | US 5879670 A | 09-03-1999 |
| | | AU 6742998 A | 22-10-1998 |
| | | BR 9809025 A | 01-08-2000 |
| | | EP 1021471 A | 26-07-2000 |
| | | US 6066315 A | 23-05-2000 |

EPO FORM P0460

Pour tout renseignement concernant cette annexe : voir Journal Officiel de l'Office européen des brevets, No.12/82